# EUROPEAN PATENT SPECIFICATION

(11) **EP 0 521 916 B1**
(45) Date of publication and mention of the grant of the patent: **17.01.1996**
(21) Application number: 91906129.1
(22) Date of filing: 19.03.1991
(51) Int. Cl.: A61K 39/39

(54) **USE OF IL-4 TO ENHANCE IMMUNE RESPONSE TO INFECTIOUS ANTIGENIC CHALLENGES**
VERWENDUNG VON IL-4 ZUR VERSTÄRKUNG DER IMMUNANTWORT AUF INFEKTIÖSE ANTIGEN-HERAUSFORDERUNGEN
UTILISATION DE L'IL-4 POUR STIMULER LA REPONSE IMMUNITAIRE AUX ATTAQUES D'ANTIGENES INFECTIEUX

(30) Priority: 21.03.1990 US 496832
(43) Date of publication of application: 13.01.1993
(73) Proprietor: SCHERING CORPORATION, Kenilworth New Jersey 07033 (US)
(72) Inventor: SCHWARTZ, Jerome 500 A East 87th Street, New York, NY 10128 (US); NASH, Claude, H., Verona, NJ 07044 (US)
(74) Representative: Ritter, Stephen David
(86) International application number: US9101733
(87) International publication number: WO9114450

(56) References cited:
- EP-A- 302 429
- EP-A- 351 876
- WO-A-87/02990
- WO-A-90/07932
- EUROPEAN JOURNAL OF IMMUNOLOGY, vol. 19, no. 4, 1989, VCH VerlagsGesmbH, Weinheim (DE); K.C. CARTER et al., pp. 779-782/
- THE LANCET, vol. I, no. 8628, 07 January 1989, London (GB); S. MEUER et al., pp. 15-18/

## Description

### BACKGROUND OF THE INVENTION

This invention relates to pharmaceutical compositions comprising IL-4 as an active ingredient for inducing an effective immune response to endemic chronic infectious agents in mammals exposed to such agents. The invention also relates to the use of IL-4 for making medicaments for inducing an effective immune response to endemic chronic infectious agents as well as to methods of inducing an effective immune response to endemic chronic infectious agents in mammals exposed to such agents by administering to said mammals an effective amount of interleukin-4.

### INTRODUCTION

Interleukin-4 [hereinafter "IL-4" but also known as B Cell Stimulatory Factor 1, (BSF-1)] was originally described by M. Howard et al. in J. Exp. Med. (1982), Vol. 155, pp. 914-23 as a T cell-derived growth factor, distinct from IL-2, which permitted long-term tissue culture of normal mouse B lymphocytes and which interacted with activated B lymphocytes to maintain the proliferation thereof for as long as 4 months. Although mixed B lymphocyte explants have been used to initiate cultures, it appears that B lymphocytes with immature phenotype are specifically enhanced by IL-4 in tissue culture. See for example C. Perchel et al., J. Immunol. (1989), Vol. 142, 1558-1568. In addition, G. Trenn et al. J. Immunol. (1988) Vol. 140, 1101-1106 discloses that IL-4 stimulates the development of cytotoxic T cells from the Lyt-2+ subpopulation of resting murine T lymphocytes H. Spits et al., J. Immunol. (1987), Vol. 139, 1142-47 discloses that IL-4 can act as a T cell growth factor on human cells in-vitro distinct from IL-2.

The mouse IL-4 gene was cloned and expressed in COS-7 cells [See T. Otsuka et al., Nuc. Acids Res. (1987), Vol. 15, 333-334. The cloned factor had all the activities in tissue culture seen for the factor purified from T cell culture supernatants. Cloning and expression of the human IL-4 gene have been described by N. Arai et al., J. Immunol. (1989), Vol. 142, 274-282 and T. Yokota et al., Proc. Natl. Acad. Sci. (1986), Vol. 83, 5844-5848 with the factor produced in COS-7 cells having similar activities to the native molecule as studied in tissue culture. As IL-4 was studied both in human and murine cell systems, additional in-vitro activities were attributed to the molecule: i) IL-4 plays an important role in the induction and regulation of IgE synthesis, a process occuring as B lymphocyte subpopulations are induced into proliferation [See S. Romagnani et al., Clin. Immunol. Immunopathol. (1989,) Vol. 50, 513-523); ii) IL-4 induces low affinity Fcε receptors (CD23) on normal human B lymphocytes in tissue culture [See T. DeFrance et al., J. Exp. Med. (1987), Vol. 165, 1459-1457]; and iii) IL-4 interacts in an extremely precise way with other lymphokines, notably interferon-γ[See R. L. Coffman et al., Immunol. Res. (1988), Vol. 102, 5-27 and S. Romagnani et al., supra] and T cells [See R. L. Coffman et al. supra, S. Romagnani et al. supra, and M. D. Widmer et al., Nature, (1987), Vol. 326, 795-98] to bring about B cell proliferation and alteration.

There is still a need to demonstrate that the reported in vitro activities of human IL-4 can be translated into an increased and enhanced immune response such as in the form of increased antibody levels to endemic chronic infectious agents in mammals exposed to such agents.

Carter et al Eur. J. Immunol. 1989, Vol. 19, 779-782, describes the ability of IL-4 to induce protective immunity to *Leishmania major* in the BALB/c mouse strain.

EP-A-0,302,429 describes a human IL-4 and states that it improves the immune response and hence is useful for the treatment and prevention of diseases which require such improvement, for example cancer, infections, AIDS, functional immune deficiency etc..

EP-A-0,351,876 describes a composition for potentiating the vaccination effect which consists of human B-cell differentiation factor (BCDF). Compositions comprising BCDF in combination with IL-1, IL-2, IL-3, IL-4, IL-, γ-IFN, GM-CSF and M-CSF are referred to.

WO 87/02990 describes a mammalian IL-4, in particular human IL-4 and the isolation of the corresponding cDNA. The use thereof in compositions for enhancing natural defence against various infections is described.

### SUMMARY OF THE INVENTION

Surprisingly, we have found that an effective immune response to endemic chronic viral infectious agents may be induced in mammals exposed or re-exposed to such agents by administering an effective amount of IL-4 to mammals, such as human beings, exposed to such agents.

Accordingly the present invention provides the use of IL-4 for the manufacture of a medicament for inducing an effective immune response to a viral infectious agent in a mammal exposed to such an agent.

### DESCRIPTION OF THE FIGURES

Figure 1 illustrates the enhancement of immune response achieved by administration of human IL-4 in an simian model (the African Green Monkey) of human viral disease (simian varicella virus) in accordance with the present invention.

Figure 2 illustrates the appearance antibodies to the human viral disease in the simian model by administring recombinant human IL-4 in accordance with the present invention.

### DETAIL DESCRIPTION OF THE INVENTION

It was the objective of the present study to translate the many reported activities of human IL-4 seen in tissue culture to a mammalian, i.e., simian model in-vivo. To evaluate the in-vivo activity of IL-4, the simian model of human varicella-zoster virus infection was chosen. In this model, African green monkeys (Cercopithecus aerthiops) were infected with a near lethal challenge of simian varicella virus (SVV). Survivors of infection developed a solid immunity to disease upon re-infection with SVV and this immunity is disclosed by E. D. Roberts et al. in, Am. J. Vet. Res. (1984), 45, 523-30, to be derived from circulating anti-SVV antibodies classically derived from fixed and/or circulating B Lymphocytes. This in-vivo mammalian model is traditionally used by those skilled in the art to evaluate potential human anti-varicella zoster antivirals and details of this mammalian mode and examples of the utility thereof have been published by K. F. Soike et al., in Antimicrob. Ag. Chemother., (1986), Vol. 29, 20-25 and in Antiviral Res., (1981), Vol. 1, 325-337. D. W. Horohov et al., J. Immunol. (1988), Vol. 141, 4217-4273 have been reported that IL-4-induced alteration of immune cells in tissue culture effects replication of influenza virus. Aspects of replication of human T cell leukemia virus type-1 have been disclosed by S. Miyatake et al., Nuc. Acids Res., (1988), Vol. 16, 6541-6566. Thus, it was appropriate to evaluate IL-4 in the SVV simian model for effects of IL-4 on antibody production and immune cell proliferation.

We have surprisingly discovered that the African green monkeys receiving an effective amount of recombinant human IL-4 produced a statistically stronger and faster immune response to the SVV upon reinfection or re-exposure thereto compared to the monkeys not receiving recombinant human IL-4.

The phrase "inducing an effective immune response to viral infectious agent in mammals exposed to such agent" includes: (1) inducing an effective level of antibodies in mammals first exposed to such viral infectious agents; (2) inducing an effective level of antibodies in mammals re-exposed to viral infectious agents; (3) enhancing an immune response in mammals re-exposed to viral infectious agents wherein the immune response by said mammal in the absence of IL-4 is not strong enough or fast enough to prevent re-occurence of dIsease; and (4) enhancing an immune response in mammals first exposed to viral infectious agents wherein such immune response in the absence of IL-4 is not strong enough or fast enough to prevent occurrence of the disease. Even though immune responses by said mammals in the absence of IL-4 is not strong or fast enough to prevent disease in said mammals, we have found effective methods which comprise administering to such mammals an amount of IL-4, preferably recombinant human IL-4, effective for each of such purposes.

The term "viral infectious agents" means those viral infectious agents which induce an immune response in a mammal including those which cause the human immune deficiency virus (HIV), i.e., the AIDS virus.

Any suitable IL-4 may be employed in the present invention. Complementary DNAs (cDNAs) for IL-4 have recently been cloned and sequenced by a number of laboratories, e.g. Yokota et al., *Proc. Natl. Acad. Sci. USA*. (1986) Vol. 83: 5894-5898 (human); Lee et al., *Proc. Natl. Acad. Sci. USA*. (1986) Vol. 83: 2061-2065 (mouse); Noma et al., *Nature* (1986) Vol. 319: 640-646 (mouse); and Genzyme Corporation, Boston, Massachusetts (human and mouse). Moreover, non-recombinant IL-4 has been purified from various culture supernatants, e.g. Grabstein et al., *J. Exp. Med*., (1985) Vol. 163: 1405-1413 (mouse); Ohara et al., *J. Immunol*., (1985) Vol. 135: 2518-2523 (mouse BSF-1).

Preferably, the IL-4 used in the present invention is human IL-4, and most preferably it is the human version with the sequence described in Yokota et al., *Proc. Natl. Acad. Sci*. USA (1986) Vol 83: 5894-5898 and PCT Patent Application No. 87/02990 published May 21, 1987 that is expressed in and isolated from E. coli (EP 0 301 835 and EP 0 342 892). The production of IL-4 from CHO cells is described in WO 91/01744. The production of IL-4 from E. coli is described in WO 91/06655. The above articles describe DNA and amino acid sequences and methods of obtaining IL-4 materials for use in the present invention.

According to this invention, mammals are administered an effective amount of an IL-4 to prevent occurrence or re-occurrence of disease caused by exposure to viral infectious agents, to increase antibody levels and/or an effective immune response to first exposure and/or re-exposure to viral agents. Such an effective amount is defined as any amount of IL-4 that will significantly increase the antibody levels with an increased count of at least 25 percent, preferably 50%, considered significant. From about 0.25 to about 15 micrograms of IL-4, preferably human IL-4 recombinantly produced from E. coli or CHO cells, per kilogram of body weight per day is preferably administered. More preferably, mammals are administered about 5 to about 15.0 micrograms of hIL-4 per kilogram of body weight per day, and most preferably mammals are administered about 5 to about 10.0 micrograms of hIL-4 per kilogram of body weight per day.

The amount, frequency and period of administration will vary depending upon factors such as the level of the neutrophil and monocyte count (e.g., the severity of the monocytopenia or granulocytopenia), age of the patient, nutrition, etc. Usually, the administration of IL-4 will be daily initially and it may continue periodically during the patient's lifetime. Dosage amount and frequency may be determined during initial screenings of neutrophil count and the magnitude of the effect of IL-4 upon the increase in antibody levels.

Administration of the dose can be intravenous, nasal, parenteral, oral, subcutaneous, intramuscular, topical, transdermal or any other acceptable method. The IL-4 can be administered in any number of conventional dosage forms. Parenteral preparations include sterile solutions or suspensions. Inhalation administration can be in the form of a nasal or oral spray, or by insuffulation. Topical dosage forms include creams, ointments, lotions, transdermal devices (e.g., of the conventional reservoir or matrix patch type) and the like.

The formulations of pharmaceutical compositions contemplated by the above dosage forms can be prepared with conventional pharmaceutically acceptable excipients and additives, using conventional techniques.

Presently, the IL-4 is preferably administered via the intravenous route. The solutions to be administered may be reconstituted lypholized powders and they may additionally contain preservatives, buffers, dispersants, etc.

Preferably, IL-4 is reconstituted with 10 millimolar citrate buffer and preservative-free sterile water with the maximum concentratin not to exceed 100 micrograms per milliliter and administered by continuous intravenous infusion or by intravenous injection. For continuous infusion, the daily dose can be added to 5 ml of normal saline and the solution infused by mechanical pump or by gravity.

The effect of IL-4 on enhancing the immune response to a viral infectious agent by e.g.increasing the antibody levels in mammals can be determined by the following test protocol.

### MATERIALS AND METHODS:

The human recombinant IL-4 used in the following two experiments was CHO cell-derived and the method of its preparation is described in WO 91/01744.

Two experiments were carried out to evaluate the effect of human recombinant IL-4 ("rHuIL-4") produced in CHO cells, on humoral antibody responses to SVV re-challenge and effect on blood cell parameters.

### DESIGN OF EXPERIMENTS:

African green monkeys which had survived initial severe disease caused by experimental infection with SVV were used in re-challenge experiments to measure the effects of IL-4. Animals received IL-4 for 15 days; serum SVV neutralizing antibodies and blood cell levels were determined on day 0 and at intervals during and following the dosing period. In the below-listed Experiment 1, 30 and 5.0 ug/kg/day B.I.D. of IL-4 produced in CHO cells was studied when administered by the I.V. route; in below-listed Experiment 2, 5.0, 1.0 and 0.25 ug/kg/day of IL-4 produced in CHO calls was studied, administered by the same regimen used in Experiment 1 was studied. Groups consisted of 4 animals distributed in each experiment so variations in neutralizing SVV antibody levels seen on day 0 were distributed equally among the groups.

### MONKEYS:

African green monkeys (Arcopithecus aethiops) obtained from Somalia were purchased as feral animals from a commercial source. All monkeys were quarantined for a minimum of 90 days before use. They were wild-caught animals of mixed sex and age; their weights ranged between 2 and 6 kg. Efforts were made to select monkeys for treatment groups so that similar sex ratios and mean weights were found in each group.

### VIRUS AND ANIMAL INOCULATION:

Virus. The simian varicells virus ("SVV") used in these studies was isolated from a naturally infected patas monkey (Erythocebus patas). This isolate was passaged in African green monkeys, and a virus stock was prepared. SVV was isolated in Vero cell cultures from peripheral blood lymphocytes from an infected monkey. The infected cultures were expanded by passages of scraped infected cells to freshly seeded Vero cultulres in a ratio of 1:3. At the fifth passage level, infected Vero cells were scraped from the surface, inoculated into culture flasks and suspended in Eagle minimum essential medium with 5% by weight of fetal bovine serum and 35% by weight sorbitol. The SVV stock was dispensed in ampoules and stored at -70°C.

Animal Inoculation. Monkeys were infected with a predetermined dilution of stock SVV which was administered as 1.5 mL transtracheally through the cricoid cartilage and 1.5 mL subcutaneously into the abdominal area. The inoculum was titrated for plaque-forming units of virus in Vero cells at the time of each experiment.

### Antibody Analysis:

The level of neutralizing antibodies to SVV was determined on day 0 of each Experiment and at indicated time intervals. Titers are expressed as the reciprocal of the highest dilution required to neutralize 80% of virus as determined in SVV plaque forming assay on Vero cells. Briefly, monkey serum samples were incubated for 60 minutes at 37°C with 100-200 plaque forming units of SVV. After incubation, the mixture was placed onto the cells, incubated for 60 minutes at 37°C and overlayed with agar. After 7 days incubation at 37°C, cells were stained and the number of SVV plaques remaining determined.

### Blood Parameter Determinations:

For each serum sample, the number of red blood cells, total number of white blood cells and subpopulations, and number of platelets per ml was determined by methodologies previously described by E.D. Roberts et al., Am. J. Vet. Res (1984), Vol 45, 523-530 and by K. F. Soivie et al in Antimicro. Ag. Chemother (1986), Vol. 29, 20-25 and Antiviral Res. (1981), Vol 1, 325-337. In addition, hemoglobin and hematocrit determinations were made using methodogies well known to those skilled in the art.

### Results:

The following two experiments were carried out to measure the effects of IL-4 produced in CHO cells on the immune response to re-infection with SVV in African green monkeys.

### Experiment 1:

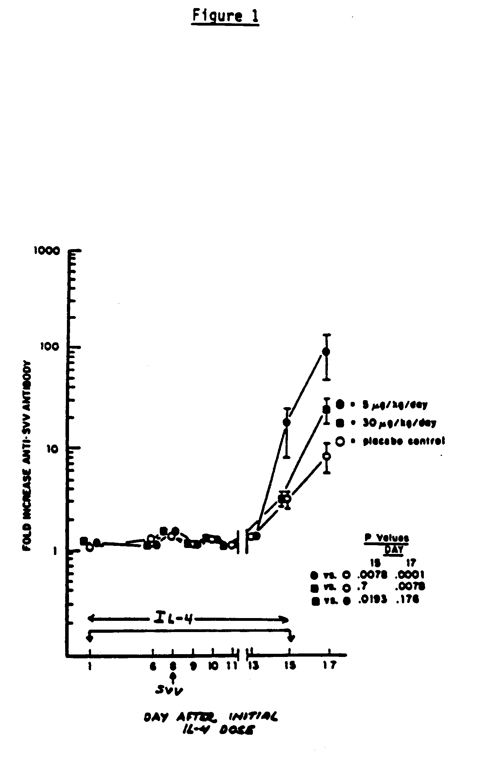

African green monkeys which survived initial severe infection with SVV were used. On rechallenge with SVV, a rapid increase in antibody was seen. Re-challenge in the presence and absence of daily I.V. of rHuIL-4 at two dose levels (5.0 and 30.0 µg/kg/day B.I.D.) and placebo were studied. Each group contained four animals and human IL-4 administration started at day 0 and continued through day 15. Animals were re-challenged with SVV on day 8 of rHuIL-4 or placebo administration. At various time intervals, blood samples were taken to monitor appearance of SVV neutralizing antibodies, white blood cell number determinations and measurement of other blood parameters. Results of the determination of the appearance of SVV neutralizing antibody are shown in Figure 1.

Both 5.0 and 30.0 µg/kg/day rHuIL-4 resulted in a statistically higher level of antibody response to SVV re-challenge. The 5.0 response was significantly higher than the 30.0 µg/kg/day. The absence of a dose-response could be due to the sharp optimal dose for IL-4 seen in tissue culture.

In addition, the calculated slopes of onset of the antibody levels were different for the animals receiving rHuIL-4, indicating that the antibodies appeared faster in these groups than in the placebo group.

### Experiment 2:

A experiment, similar to Experiment 1 was carried out. Three dose levels of rHuIL-4 derived from CHO cells were employed. 5.0, 1.0 and 0.25 µg/kg/day B.I.D. were administered by the I.V. route over a 15 day period in a similar fashion to Experiment 1. There were 4 animals per group.

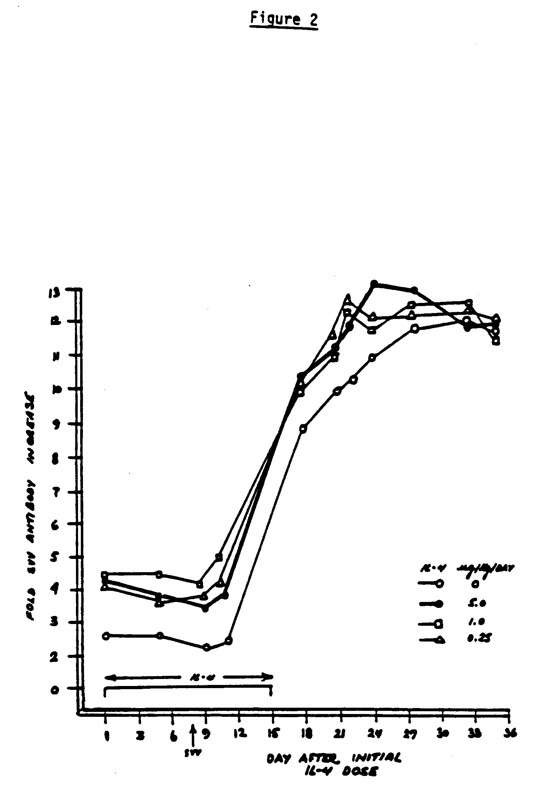

Results of the appearance of antibodies to SVV are shown in Figure 2. Although there were no statistically significant differences at the lower dose levels, at 5.0 µg there was a trend to higher antibody levels, at day 22. The lower dose levels (1.0 and 0.25 µg/kg/day) were not effective in enhancing antibody response. Both Experiments 1 and 2 show enhanced antibody production occurs at doses of 5.0 µg/kg/day and above in monkeys re-exposed to an endemic chronic infection such as SVV.

In view of the results in the above Experiments, it is expected that IL-4, preferably rHuIL-4, would enhance an immune response in mammals after first or primary exposure to a viral infection wherein immune response by said mammals is not strong enough or fast enough to prevent occurrence of disease.

## Claims

1. The use of IL-4 for the preparation of a medicament for inducing an effective immune response to a viral infectious agent in a mammal exposed to such agents.

2. The use according to Claim 1 wherein the viral infectious agent is a varicella virus.

3. The use according to any preceding claim wherein the IL-4 is human IL-4.

4. The use according to Claim 3 wherein the IL-4 is E.coli-derived recombinant IL-4.

## Patentansprüche

1. Verwendung von IL-4 zur Herstellung eines Medikaments zum Induzieren einer wirksamen Immunantwort auf einen viralen Infektionserreger bei einem Säuger, der solchen Erregern ausgesetzt ist.

2. Verwendung gemäß Anspruch 1, wobei der virale Infektionserreger ein Varicella-Virus ist.

3. Verwendung gemäß einem der vorangehenden Ansprüche, wobei es sich bei dem IL-4 um Human-IL-4 handelt.

4. Verwendung gemäß Anspruch 3, wobei das IL-4 von *E. coli* abgeleitetes rekombinantes IL-4 ist.

## Revendications

1. Utilisation de IL-4 pour la préparation d'un médicament pour induire une réponse immunitaire efficace vis-à-vis d'un agent infectieux viral chez un mammifère exposé à de tels agents.

2. Utilisation selon la revendication 1 où l'agent infectieux viral est un virus de la varicelle.

3. Utilisation selon toute revendication précédente où IL-4 est IL-4 humaine.

4. Utilisation selon la revendication 3 où IL-A est IL-A recombinante dérivée de E. coli.
